(19) Europäisches Patentamt
European Patent Office
Office européen des brevets

(11) Numéro de publication: **0 017 568**
**B1**

(12) **FASCICULE DE BREVET EUROPEEN**

(45) Date de publication du fascicule du brevet: **22.12.82**

(21) Numéro de dépôt: **80400413.3**

(22) Date de dépôt: **28.03.80**

(51) Int. Cl.³: **C 07 C 143/14,** C 07 C 143/72, C 11 D 1/92, A 62 D 1/00

# (54) Sulfobétaïnes fluorées et applications de leurs propriétés tensio-actives à des compositions extinctrices.

(30) Priorité: **06.04.79 FR 7908749**

(43) Date de publication de la demande: **15.10.80 Bulletin 80/21**

(45) Mention de la délivrance du brevet: **22.12.82 Bulletin 82/51**

(84) Etats contractants désignés: **BE CH DE FR GB NL SE**

(56) Documents cités: **FR - A - 2 122 918**

(73) Titulaire: **P C U K PRODUITS CHIMIQUES UGINE KUHLMANN**
**Service Propriété Industrielle Tour Manhattan**
**F-92087 Paris La Defense 2 Cédex 21 (FR)**

(72) Inventeur: **Bertocchio, Rene**
**31, rue des Vallières**
**F-69390 Vourles par Vernaison (FR)**
Inventeur: **Foulletier, Louis**
**5, Chemin Croix-Berthet**
**F-69600 Oullins (FR)**
Inventeur: **Lantz, Andre**
**Domaine de la Hêtraie**
**F-69390 Vernaison (FR)**

(74) Mandataire: **Bernard, Nicole et al,**
**PCUK Produits Chimiques Ugine Kuhlmann**
**Service Propriété Industrielle Tour Manhattan - Cedex 21**
**F-92087 Paris la Defense 2 (FR)**

**EP 0 017 568 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Sulfobétaïnes fluorées et applications de leurs propriétés tensio-actives à des compositions extinctrices

La présente invention concerne une nouvelle classe de tensio-actifs fluorés ampholytes caractérisés par le fait qu'ils contiennent dans une même molécule, un radical perfluoré aliphatique, un groupe ammonium quaternaire et un groupe anionique sulfonique lié chimiquement au groupe ammonium quaternaire par l'intermédiaire d'un reste bivalent. La formule générale de ces dérivés, qui sont des sulfobétaïnes peut être schématisée par:

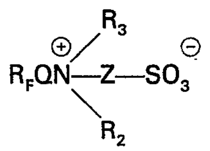

(I)

où $R_F$ représente une chaîne perfluorée aliphatique $C_nF_{2n+1}$, droite ou ramifiée, n étant un nombre entier compris entre 1 et 20 et Q et Z représentent des groupes organiques bivalents de liaison. Les groupes $R_2$ et $R_3$ sont des radicaux alkyles contenant en général 1 à 6 Atomes de carbone.

Des composés particulièrement efficaces et répondant à cette formule générale peuvent être représentés par la formule suivante:

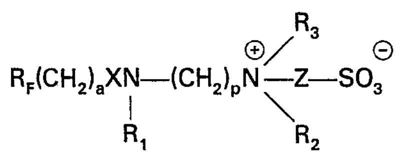

(II)

Dans cette formule les groupes $R_F$, $R_2$, $R_3$ et Z ont les mêmes significations que ci-dessus et les autres termes ont les significations suivantes:

— a et p sont des nombres entiers jusqu'à 10
— X est —CO— ou —$SO_2$—
— $R_1$ est un atome d'hydrogène ou un reste alkyle contenant 1 à 6 atomes de carbone

Dans cette même formule Z représente un groupe organique bivalent de liaison. Z peut en particulier représenter un groupe —$(CH_2)_q$— où q est un numbre entier compris entre 1 et 10, de préférence entre 1 et 3, mais Z peut aussi représenter un groupe plus complexe, en particulier des radicaux polyméthylène substitués soit par des groupes alkyl, soit par des groupes fonctionnels tels que OH ou COOH.

Parmi les composés représentatifs de l'invention il y a ainsi:

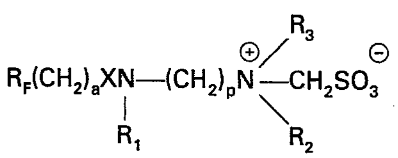

(III)

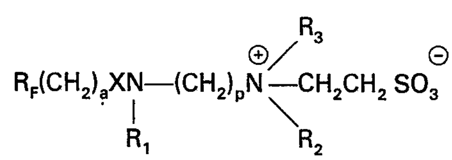

(IV)

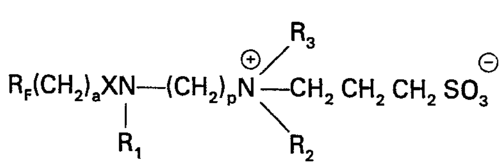

(V)

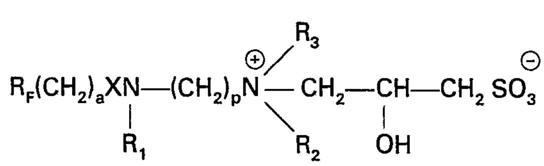

(VI)

On connaît de nombreux tensioactifs fluorés amphotères. Dans les USP. 3.258.423 et 3.562.156, est par exemple décrit le composé suivant:

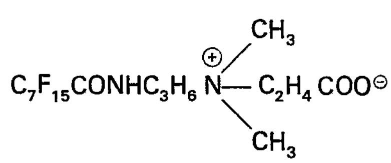 .

Les brevets français 2.088.699, 2.088.941 et 2.084.888 décrivent les produits de formules suivantes:

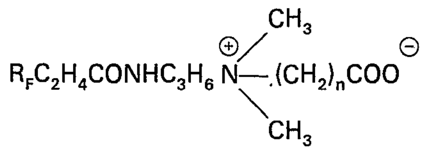

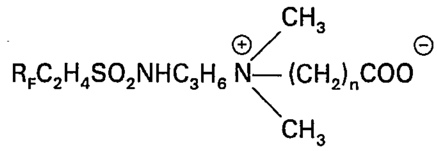

Ces produits ampholytes ont trouvé de nombreuses applications, dans des domaines très variés, mais en particulier dans le domaine des agents extincteurs (voir par exemple les brevets français 2.185.668 et 2.308.674).

Ces tensioactifs ampholytes sont cependant tous des ampholytes carboxyliques c'est-à-dire des produits où le groupement anionique est l'anion carboxylique $CO_2^-$. D'autres tensioactifs fluorés ampholytes ont été décrits. Ainsi le brevet français 2.373.551 revendique des composés où le groupement anionique est l'anion phosphoreux et le brevet français 2.122.918 des produits oú l'anion est le groupement sulfonique $SO_3^-$ comme par exemple:

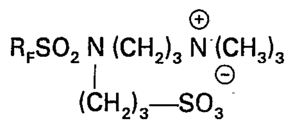

Les sulfobétaïnes correspondant à cette dernière publication sont caractérisées par le fait que les groupements ammonium quaternaire et sulfonique contenus dans leur molécule sont tous les deux terminaux. Les sulfobétaïnes, objet de notre invention, et représentées par les formules (I) et (II) n'ont pas d'ammonium quaternaire terminal, ce groupement ammonium quaternaire étant au contraire lié, par l'intermédiaire d'un groupe organique bivalent, aussi bien au radical perfluoré, qu'à la partie sulfonique de la molécule. Les sulfobétaïnes de l'invention peuvent être considérées comme des tensioactifs amphotères linéaires et sont obtenues selon des procédés de fabrication différents de ceux nécessaires pour la préparation des sulfobétaïnes non linéaires décrits dans le brevet français 2.122.918 cité ci-dessus.

Les sulfobétaïnes répondant à la formule (II) peuvent être obtenues par quaternisation des amines de formule:

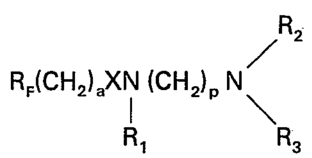 (VII)

où $R_F$, X, $R_1$, $R_2$, $R_3$, a et p ont les mêmes significations que ci-dessus.

Les amines (VII) c'est-à-dire:

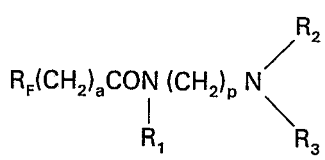 et 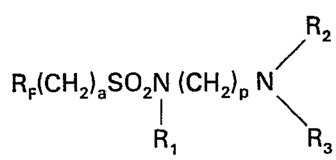

sont déjà connues et peuvent être préparées par exemple selon les procédés des brevets français n° 2.086.904 et 2.088.594 en particulier par réaction des diamines

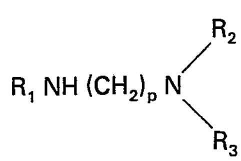

avec les chlorures d'acides sulfonique

$$R_F(CH_2)_aSO_3H$$

ou carboxylique

$$R_F(CH_2)_aCOOH$$

Pour des raisons de disponibilité de matières premières on préfère les amines de formule (VII) avec

a = 0 ou 2

$R_1$ = H ou méthyl

$R_2$ et $R_3$ = méthyl

p = 2 ou 3

et tout particulièrement les produits suivants:

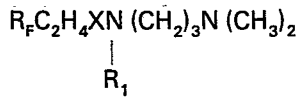

où X représente CO ou $SO_2$ et $R_1$ l'hydrogène ou un radical méthyl.

Les sulfobétaïnes peuvent être obtenues à partir des amines de formule (VII) par réaction avec des sels d'acides halogénosulfoniques ou avec des sultones. D'autres procédés de quaternisation sont aussi utilisables dont certains sont indiqués ci-dessous. Les sulfobétaïnes de formule (III) dans lesquelles Z = $-CH_2-$ peuvent ainsi être obtenues par réaction de l'amine avec un sel de l'acide chloro - ou bromo méthane sulfonique.

Les sulfobétaïnes de formule (IV) c'est-à-dire les dérivés de formule générale (II) avec Z = $-CH_2-CH_2-$ peuvent être obtenues par réaction de l'amine (VII) avec un sel de l'acide chloro-2 ou bromo-2 éthane sulfonique par exemple selon la réaction suivante:

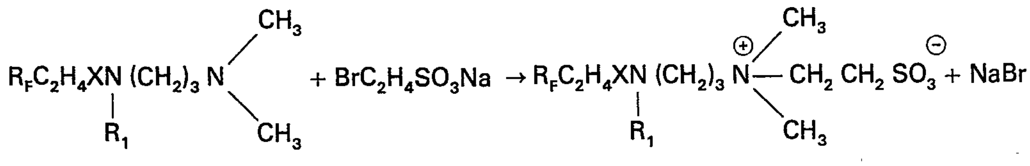

Ce même produit peut également être obtenu selon une méthode (J. Org. Chem. 1961, *26*, 4521) consistant à quaterniser l'amine avec le dibromoéthane et à faire réagir le monosel formé avec du sulfite de sodium

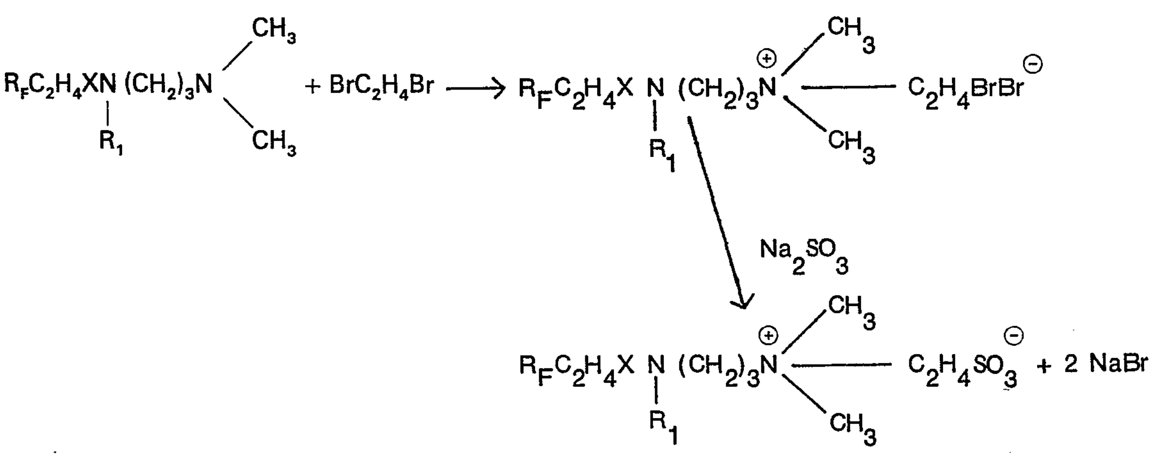


Les bétaïnes de formule (V) peuvent être obtenues selon des méthodes connues par réaction de l'amine (VII) soit avec la propane sultone

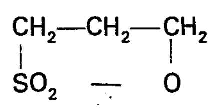

soit avec un sel de l'acide chloro-3 propane sulfonique. Les mêmes produits peuvent aussi être obtenus selon une méthode décrite dans J. of American Oil Chem. Soc. 1977, *54*, p. 294, consistant à quaterniser l'amine avec du chlorure d'allyle et à faire réagir ce sel avec du bisulfite de sodium.

Les bétaïnes de formule (VI) peuvent être préarées selon une méthode décrite dans J. of American

Oil Chem. Soc. 1976, *53*, 60 et schématisées dans le cas des produits de l'invention par les réactions suivantes:

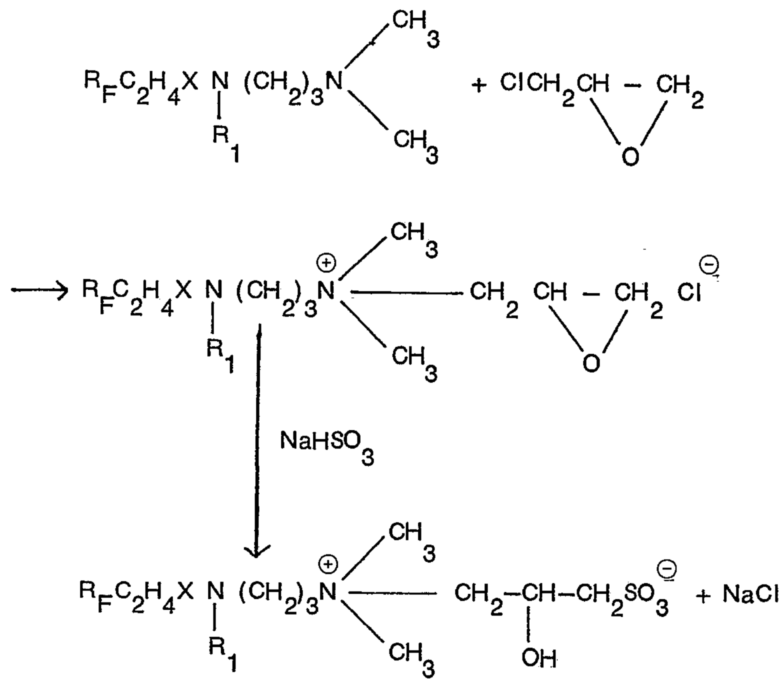


L'ordre de ces réactions peut aussi être inversé en faisant réagire tout d'abord l'épichlorhydrine avec du bisulfite de sodium et en faisant réagir le produit formé c'est-à-dire

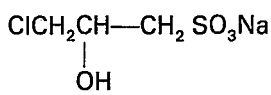

avec l'amine.

Les sulfobétaïnes de l'invention sont d'excellents agents tensioactifs. Toutefois, ces produits étant peu solubles dans l'eau (solubilité en générale inférieure à 0,1% à température ambiante), il peut être intéressant pour certaines applications d'utiliser ces sulfobétaïnes en mélange avec d'autres tensioactifs, fluorés ou non, en particulier avec des tensioactifs non ioniques qui permettent d'augmenter la solubilité des produits fluorés. Ces produits présentent aussi la particularité d'être plus solubles, même en présence de co-tensioactifs, dans des solutions aqueuses salines, que dans l'eau douce. En vue d'obtenir des solutions relativement concentrées en produits de l'invention, il est parfois souhaitable d'ajouter une certaine quantité d'alcools légers, de glycols ou d'éthers de glycols.

Ces propriétés tensioactives sont illustrées par le tableau ci-dessous qui donne les valeurs de tensions superficielles à 20°C de quelques sulfobétaïnes de l'invention, à la concentration de 0,1%, en présence de 0,3% d'un tensioactif non ionique non fluoré, par exemple octyl phénol éthoxylé à 10 O E vendu sous la marque Triton X 100 par la société ROHM & HAAS, dans de l'eau der mer synthétique correspondant à la composition suivante:

1,1% $MgCl_2$, 6 $H_2O$

0,16% $CaCl_2$, 2 $H_2O$

0,4% $Na_2SO_4$

2,5% NaCl

95,84% eau distillée

La tension superficielle de cette solution contenant uniquement 0,3% de Triton X 100 sans addition de produits fluorés est de 26 dynes/cm à 20°C.

| Tensions superficielles à 20 °C en dynes/cm de solutions d'eau de mer contenant 0,1 % de sulfobétaïne et 0.3 % de Triton × 100 | |
|---|---|
| 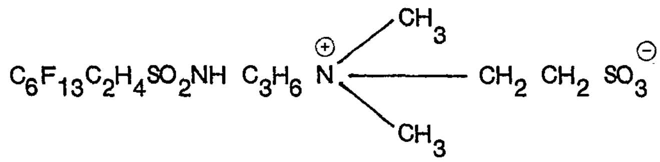 | 15,3 |
| 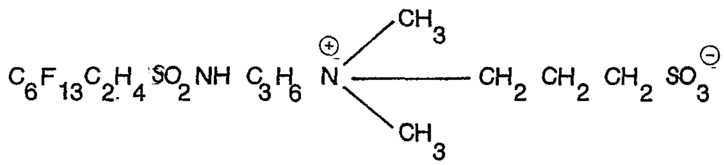 | 14,5 |
| 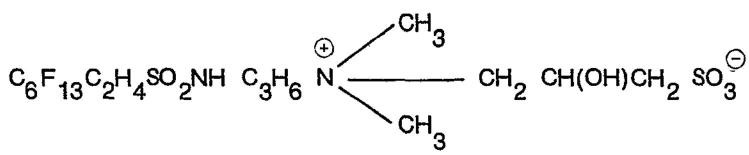 | 14,5 |
| 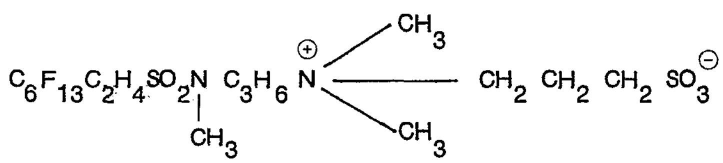 | 16,8 |
| 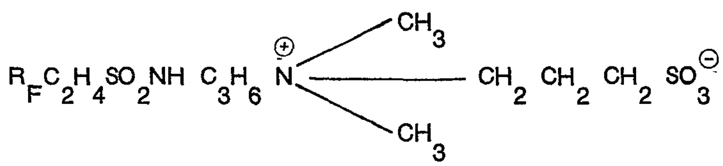 | 13,8 |
| $R_F$ = mélange $C_6F_{13}$ à $C_{18}F_{37}$<br>correspondant à l'exemple n° 5 | |

Les bonnes propriétés tensioactives des sulfobétaïnes peuvent aussi être mises en évidence par leur faculté de former un film à la surface d'un hydrocarbure lorsqu'on dépose une solution aqueuse de tensioactif sur l'hydrocarbure. Il est connu par les USP. 3.258.423 et 3.562.156 que les solutions aqueuses de tensioactif qui forment un film à la surface des hydrocarbures peuvent être utilisées comme agents extincteurs. Cette propriété peut être caractérisée par la mesure de la vitesse d'étalement. Cette vitesse peut être déterminée de la façon suivante:

Une coupelle en verre de 120 mm de diamètre est remplie à moitié avec du cyclohexane. On dépose 0,1 ml de la solution tensioactive au centre de la nappe hydrocarbonée. La différence de pouvoir réflecteur permet de suivre la progression du film fluoré et de mesurer ainsi le temps nécessaire pour obtenir le recouvrement de toute la surface. Ce test d'étalement peut être réalisé avec des solutions de tensioactifs à différentes concentrations dans l'eau douce et dans l'eau de mer.

Un certain nombre de sulfobétaïnes ont subi ce test d'étalement sur cyclohexane, en mélange avec un tensioactif non ionique non fluoré, le Triton X 100.

On a préparé tout d'abord les solutions suivantes:

A) 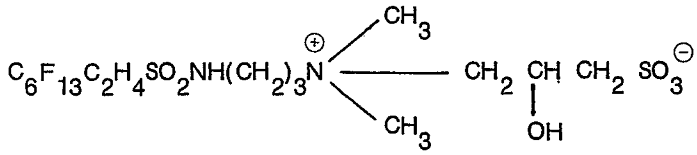 5,5 g

Triton X 100 14,4 g

Ethanol 36 g

Eau 74,1 g

B) 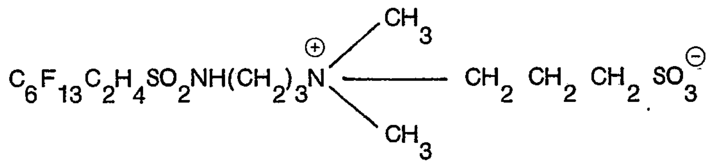 5,35 g

Triton X 100 14,4 g

Ethanol 36 g

Eau 74,25 g

C) 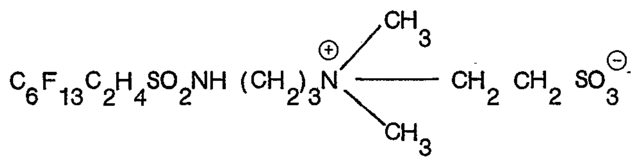 5,20 g

Triton X 100 14,4 g

Ethanol 36 g

Eau 74,4 g

D) 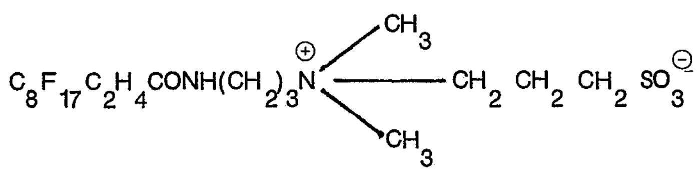 4,5 g

Triton X 100 14,4 g

Ethanol 6 g

Eau 75,10 g

Les solutions A, B, C et D ont ensuite été diluées soit avec de l'eau douce, soit avec de l'eau de mer synthétique de façon à avoir une concentration en fluor de 520 mg par litre et on a déterminé les vitesses d'étalement de ces solutions. Les résultats suivants ont été obtenus:

| | Vitesse d'étalement | |
|---|---|---|
| | Solution correspondant à 520 mg litre de fluor dans | |
| | Eau douce | Eau de mer |
| Solution A | | 2,5 sec. |
| „ B | 3 secondes | 2 „ |
| „ C | 8,5 „ | 3,5 „ |
| „ D | 7 „ | 15 „ |

La solution A dans l'eau douce n'est pas entièrement soluble à la concentration utilisée pour ces essais et la vitesse d'étalement n'a pas été mesurée. Il est encore possible d'obtenir un film avec des quantités plus faibles de produit fluoré. Ainsi, les produits B et D, dilués dans l'eau douce à 340 mg/litre de fluor ont des vitesses d'étalement de 6,5 secondes. Les résultats d'étalement sont en général meilleures en milieu salin qu'en milieu eau douce. Ainsi le produit B à la concentration de 170 mg de fluor par litre étale en 18,5 secondes en milieu eau de mer et ne donne plus qu'un étalement partiel en milieu eau douce. Il en est de même dans le cas du produit C qui étale en 9 secondes à 340 mg/litre de fluor en milieu eau de mer et n'étale plus que partiellement en eau douce.

Les exemples suivants illustrent l'invention sans toutefois la limiter.

Exemple 1

A une solution de 57,6 g de $C_8F_{17}C_2H_4CONHC_3H_6N(CH_3)_2$, dans 85 ml de chloroforme, on introduit sous agitation en maintenant la température à 20°C, 13 g de propane sultone dissoute dans 30 ml de chloroforme. L'introduction de la propane sultone a été réalisé en 30 minutes, puis le mélange réactionnel a été maintenu sous agitation pendant 4 heures. Un précipité blanc se forme au cours de cette réaction. Par filtration du mélange réactionnel après refroidissement dans de la glace, lavage du précipité avec 50 ml de chloroforme et séchage sous vide on a récupéré 62 g d'un produit fondant avec décomposition à 155°C et identifié par examen IR et RMN comme étant

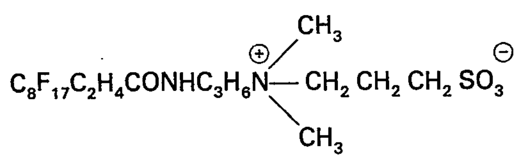

Le rendement de cette opération a été de 89%.

Une solution aqueuse à 1.000 ppm de ce produit en présence de 3.000 ppm de Triton X 100 a une tension superficielle de 15,3 dynes/cm à 20°C. A la même température une solution aqueuse de 3.000 ppm de Triton X 100 a une tension superficielle de 26 dynes/cm.

Exemple 2

Dans un réacteur de 250 ml on introduit 51,2 g de

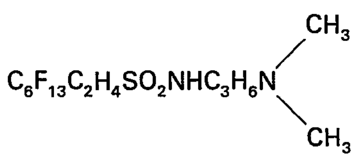

et 100 ml de chloroforme.

Après dissolution complète, on introduit à température ambiante, en une heure, 13,4 g de propane sultone. On maintient sous agitation pendant 12 heures puis les produit solide formé est filtré, lavé avec 50 ml de chloroforme et séché sous vide.

On récupère ainsi 58 g d'un produit solide blanc, fondant avec décomposition à partir de 200—220°C, identifié comme étant:

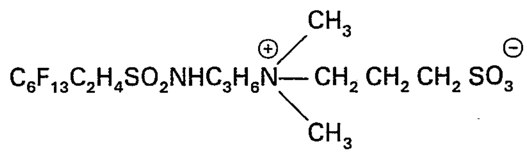

Exemple 3

Dans un réacteur de 250 ml on a introduit 51,2 g de

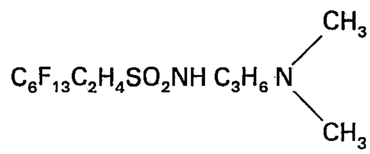

100 ml d'éthanol à 95% et 22,1 g de $Br—C_2H_4SO_3Na$ et on a chauffé le mélange à reflux, sous agitation. Au bout de 24 heures, il restait encore 6,5% d'amine non transformée. Le chauffage à reflux a été poursuivi pendant 8 heures ce qui a permis d'arriver à une teneur de 1,5% d'amine de départ non transformée. Le mélange a ensuite été filtré à chaud et par évaporation du filtrat on a obtenu 70 g d'un produit pulvérulent blanc identifié comme étant

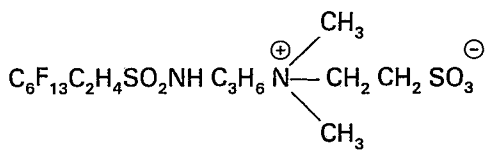

Ce produit contient 13,4% de bromure de sodium qui peut être éliminé par lavage à l'eau mais le produit brut convient pour la majeure partie des applications.

Exemple 4

Selon le mode opératoire de l'exemple 2, on a engagé 52,6 g de

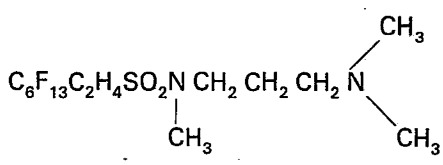

et on a obtenu 62 g de

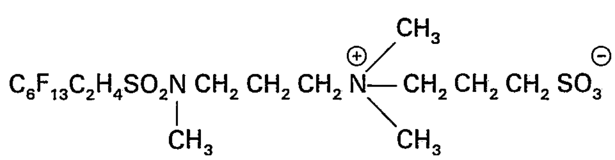

Ce produit se présente sous forme d'une poudre blanche, se décomposant à partir de 200—220°C.

Exemple 5

Selon le mode opératoire de l'exemple 2, on a engagé 56 g de

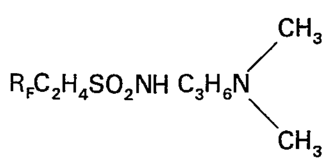

Ce produit est un mélange des différents homologues contenant:

2% de produit avec $R_F = C_4F_9$

50% de produit avec $R_F = C_6F_{13}$

28% de produit avec $R_F = C_8F_{17}$

12% de produit avec $R_F = C_{10}F_{21}$

5% de produit avec $R_F = C_{12}F_{25}$

2% de produit avec $R_F = C_{14}F_{29}$

0,6% de produit avec $R_F = C_{16}F_{33}$

0,2% de produit avec $R_F = C_{18}F_{37}$

Après réaction avec la propane sultone, filtration et séchage, on a obtenu 60 g d'un produit pulvérulent et identifié par examen IR et RMN comme étant

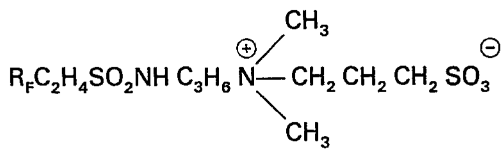

Exemple 6

Dans un réacteur de 250 ml contenant 50 g d'épichlorhydrine

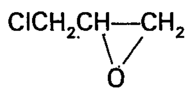

on introduit en une heure, en maitenant la température à 20°C par refroidissement, une solution contenant 64 g de $NaHSO_3$, 25 g de $Na_2SO_3$ et 150 g d'eau. Cette solution a été obtenue par dissolution de sulfite de sodium dans une solution de bisulfite de sodium à 30%. Après l'introduction on maintient encore 2 heures sous agitation à température ambiante, puis on récupère par filtration et séchage sous vide 95 g d'un solide cristallisé blanc correspondant à $ClCH_2(OH)\ CH_2SO_3Na$.

On introduit ensuite 21,6 g de ce produit dans un réacteur contenant 51,2 g de

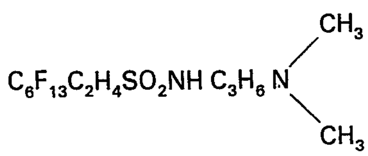

100 ml d'éthanol et 20 ml d'eau.

Le mélange est porté à l'ébullition à reflux sous agitation et on contrôle la disparition de l'amine par dosage acidimétrique. Après 15 heures de chauffage il reste encore 15% d'amine non transformée, après 22 heures 6,6% et après 34 heures 3%. L'opération est alors arrêtée et on récupère par évaporation sous vide 70 g d'un produit pulvérulent blanc correspondant à la formule suivante:

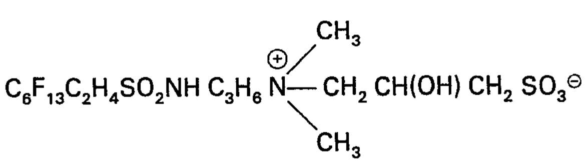

Le produit brut obtenu selon ce procédé contient 8,2% de chlorure de sodium. Il peut être purifié par dissolution dans de l'éthanol absolu, filtration du chlorure de sodium et évaporation à sec de l'éthanol.

**Revendications**

1. Composés répondant à la formule:

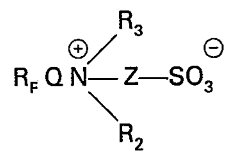

dans laquelle $R_F$ représente une chaîne perfluorée aliphatique $C_nF_{2n+1}$, droite ou ramifiée, n étant un nombre entier compris entre 1 et 20, Q représente un groupe organique bivalent de liaison, Z est un groupe $—(CH_2)_q—$ où q est un nombre entier compris entre 1 et 10, ou bien un radical polyméthylène substitué soit par des groupes alkyle, soit par des groupes fonctionnels, et $R_2$ et $R_3$ sont des radicaux alkyle contenant de 1 à 6 atomes de carbone.

2. Composés selon la revendication 1 répondant à la formule:

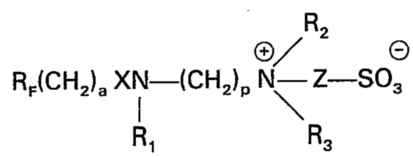

dans laquelle les groupes $R_F$, $R_2$, $R_3$ et Z ont les mêmes significations que dans la revendication 1, a et p sont des nombres entiers jusqu'à 10, X est —CO— ou $—SO_2—$ et $R_1$ est un atome d'hydrogène ou un reste alkyle contenant de 1 à 6 atomes de carbone.

3. Composés selon la revendication 2 dans lesquels a est égal à 2, p est égal à 3, $R_1$ est l'hydrogène ou un radical méthyle, $R_2$ et $R_3$ sont des radicaux méthyles.

4. Composés selon l'une des revendications 2 ou 3 dans lesquels Z est un radical alkylène inférieur $—(CH_2)_q—$ où q représente un nombre entier compris entre 1 et 3.

5. Composés selon l'une des revendications 1 à 3 dans lesquels Z est un radical $—CH_2—CH(OH)—CH_2—$.

6. Application des composés des revendications 1 à 5 comme agents tensioactifs.

7. Application des composés des revendications 1 à 5 comme agents tensioactifs en présence d'autres agents tensioactifs fluorés ou non et en présence d'électrolytes.

8. Application des composés des revendications 1 à 5, comme agents extincteurs, éventuellement en association avec d'autres tensioactifs.

**Patentansprüche**

1. Verbindungen der Formel:

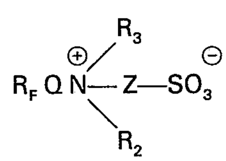

in der $R_F$ eine geradkettige oder verzweigte perfluorierte aliphatische Kette $C_nF_{2n+1}$ bedeutet, wobei n eine ganze Zahl von 1 bis 20 ist, Q eine zweiwertige organische Bindegruppe bedeutet, Z die Gruppierung $—(CH_2)_q—$, in der q eine ganze Zahl von 1 bis 10 ist, oder einen mit Alkylresten oder funktionellen Gruppen substituierten Polymethylenrest bedeutet und $R_2$ und $R_3$ Alkylreste mit 1 bis 6 Kohlenstoffatomen sind.

2. Verbindungen nach Anspruch 1 der allgemeinen Formel

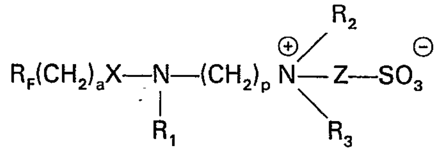

in der die Reste $R_F$, $R_2$, $R_3$ und Z die gleiche Bedeutung wie im Anspruch 1 haben, a und p ganzae Zahlen bis 10 sind, X —CO— oder $—SO_2—$ ist und $R_1$ ein Wasserstoffatom oder eine Alkylrest mit 1 bis 6 Kohlenstoffatomen ist.

3. Verbindungen nach Anspruch 2, in denen a gleich 2 p gleich 3, $R_1$ Wasserstoff oder ein

Methylrest ist, und $R_2$ und $R_3$ Methylreste sind.

4. Verbindungen nach einem der Ansprüche 2 oder 3, in denen Z ein niederer Alkylenrest $—(CH_2)_q—$ ist, in dem q eine ganze Zahl von 1 bis 3 bedeutet.

5. Verbindungen nach einem der Ansprüche 1 bis 3, in denen Z ein $—CH_2—CH(OH)—CH_2—$Rest ist.

6. Verwendung der Verbindungen der Ansprüche 1 bis 5 als oberflächenaktive Stoffe.

7. Verwendung der Verbindungen der Ansprüche 1 bis 5, als oberflächenaktive Stoffe in Gegenwart anderer gegebenenfalls fluorierter oberflächenaktiver Stoffe in Gegenwart von Elektrolyten.

8. Verwendung der Verbindungen der Ansprüche 1 bis 5, als Feuerlöschmittel, gegebenenfalls in Verbindung mit anderen oberflächenaktiven Substanzen.

**Claims**

1. Compounds corresponding to the formula:

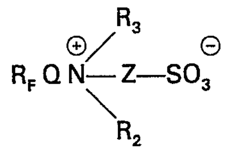

in which $R_F$ represents a linear or branched, perfluorinated aliphatic chain $C_nF_{2n+1}$, n being an integer between 1 and 20, Q represents a divalent organic linking group, Z is a group $—(CH_2)_q—$, in which q is an integer between 1 and 10, or alternatively a polymethylene radical substituted either by alkyl groups or by functional groups, and $R_2$ and $R_3$ are alkyl radicals containing from 1 to 6 carbon atoms.

2. Compounds according to Claim 1, corresponding to the formula

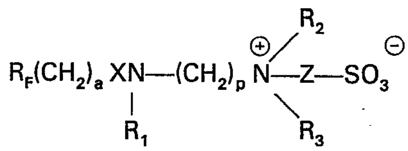

in which the groups $R_F$, $R_2$, $R_3$ and Z have the same meanings as in Claim 1, a and p are integers up to 10, X is $—CO—$ or $—SO_2—$ and $R_1$ is a hydrogen atom or an alkyl radical containing from 1 to 6 carbon atoms.

3. Compounds according to Claim 2 in which a is equal to 2, p is equal to 3, $R_1$ is hydrogen or a methyl radical and $R_2$ and $R_3$ are methyl radicals.

4. Compounds according to one of Claims 2 or 3 in which Z is a lower alkylene radical $—(CH_2)_q—$ in which q represents an integer between 1 and 3.

5. Compounds according to one of Claims 1 to 3 in which Z is a radical $—CH_2—CH(OH)—CH_2—$.

6. Application of the compounds of Claims 1 to 5 as surface-active agents.

7. Application of the compounds of Claims 1 to 5 as surface-active agents, in the presence of other fluorinated or non-fluorinated surface-active agents and in the presence of electrolytes.

8. Application of the compounds of Claims 1 to 5 as extinguishing agents, optionally in association with other surface-active agents.